# EUROPÄISCHE PATENTSCHRIFT

(11) **EP 2 479 252 B1**
(45) Veröffentlichungstag und Bekanntmachung des Hinweises auf die Patenterteilung: **30.10.2013**
(21) Anmeldenummer: 11150772.9
(22) Anmeldetag: 12.01.2011
(51) Int. Cl.: C12M 1/02, F16L 3/18, F16L 3/20, F16L 3/223

(54) **Rohrhalterung zum Haltern eines Rohrabschnitts und die Rohrhalterung aufweisender Fermenter**
Pipe holder for holding a pipe section and fermenter comprising the pipe holder
Crochet de tuyau destiné à tenir une section de tuyau et fermenteur comprenant un crochet de tuyau

(43) Veröffentlichungstag der Anmeldung: 25.07.2012
(73) Patentinhaber: Thöni Industriebetriebe Gesellschaft m.b.H., 6410 Telfs (AT)
(72) Erfinder: Krismer, Michael, 6500, Landeck (AT); Gruber, Thomas, 6410, Telfs (AT); Lechleitner, Paul, 6500, Landeck (AT)
(74) Vertreter: Dilg, Haeusler, Schindelmann Patentanwaltsgesellschaft mbH

(56) Entgegenhaltungen:
- DE-A1- 2 429 240
- DE-A1- 2 504 222
- DE-A1-102008 030 195
- DE-U1- 8 806 570
- DE-U1- 20 300 794
- DE-U1- 29 908 173
- DE-U1-202007 014 168
- DATABASE WPI Week 198719 Thomson Scientific, London, GB; AN 1987-134228 XP002642638, -& SU 1 257 349 A (KAMSK CABLE WKS) 15. September 1986 (1986-09-15)

## Beschreibung

### Gebiet der Erfindung

Die vorliegende Erfindung betrifft eine Rohrhalterung zum Haltern eines Rohrabschnitts und einen Fermenter, welcher die Rohrhalterung aufweist. Insbesondere betrifft die vorliegende Erfindung eine Rohrhalterung zum Haltern eines Heizungsrohrabschnitts, welcher zum Heizen eines Innenraums eines Fermenters zum Erzeugen von Biogas mittels der Rohrhalterung verschiebbar gehaltert ist.

### Hintergrund der Erfindung

Zum Fermentieren von biologischem oder organischem Material innerhalb eines Innenraums eines Fermenters mag das biologische Material geheizt werden. Dazu können an einer Innenseite einer Fermenterwand Heizungsrohre installiert sein. Aufgrund des sich in dem Innenraum des Fermenters befindenden und bewegten biologischen Materials sind die Heizungsrohre mechanischen und auch korrosiven Belastungen ausgesetzt. Weiterhin können zeitliche Temperaturänderungen auftreten, welche zu Spannungen und weiteren mechanischen Belastungen der Heizungsrohre führen.

DE 203 00 794 U1 offenbart eine Fermenterheizung mit einer vertikal an der Fermenterwand zu befestigenden Montageschiene, welche zur Aufnahme der Heizleitungen Aussparungen aufweist, wobei eine Abdeckleiste zur sicheren Fixierung der Heizleitungen vorgesehen ist.

DE 20 2007 014 168 U1 offenbart eine Halterung für Heizungsrohre mit einer Heizungsrohrführung, einem Abstandshalter und einem Befestigungselement, wobei der Abstandshalter überwiegend aus organischem Material besteht und wobei die Heizungsrohrführung mittels des vorzugsweise senkrecht dazu angeordneten Abstandshalters von einer Behälterwand beabstandet angeordnet ist.

GB 2 387 889 A offenbart ein wandmontiertes Rohrhalterungssystem, wobei ein Element an einer Wand fixiert werden kann und dieses Element Aussparungen zum Anbringen von Rohrhalteelementen aufweist, wobei die Rohrhalteelemente mit dem an der Wand befestigten Element einrasten oder einschnappen.

Dokument DE 24 29 240 offenbart ein Fassadenelement mit als Rohrträger dienender Platte, in welcher Langlöcher zur Befestigung an einem vorspringenden Mauerteil vorgesehen sind. Dabei sind in der Platte für die Rohrleitungen Anschweißhülsen eingesetzt. In diese Anschweißhülsen sind, gegen Verschiebung durch nach innen umgebogene Lappen an den Anschweißhülsen gesichert, Gleithülsen eingesetzt, durch die über Rollringe abgestützt, die mit einem Isoliermantel um den Isolierstoff versehene Rohrleitung hindurchgeführt ist.

Dokument SU 125 73 49 offenbart eine Rohrhalterung, welche einen Winkelabschnitt mit einer Öffnung für einen Finger aufweist, wobei der Finger einen Sitz für ein Rohr aufweist, welches mit einem U-Bolzen daran gesichert ist.

Dokument DE 10 2008 030 195 A1 offenbart eine Fördervorrichtung zum Fördern von Medien mit einem Ausleger und einer vom Ausleger getragenen Rohrleitung mit mindestens einem Rohrgelenk, wobei Rohrabschnitte seitlich und entlang ihrer Achsen dadurch verschiebbar sein können, dass sie durch Halterungen in Form von Längslöchern geführt sind.

Dokument DE 88 06 570 U1 offenbart eine Haltevorrichtung für ein Schiebestück mit Trageelementen für Rohre, wobei eine Gleitmuffe ein Schiebestück axial verschieblich führt, wobei das Schiebestück mit Rohrschellen verbunden ist, wobei die Rohrschellen ein Heizungs- oder ein Wasserleitungsrohr umspannen.

Dokument DE 25 04 222 offenbart eine Vorrichtung zum Aufhängen von Rohrleitungen, welche eine Längsverschiebung der Rohre als auch eine Querverschiebung erlaubt.

Dokument DE 299 08 173 U1 offenbart eine Anordnung zum Auflagern von Kanälen für Bioreaktoren, wobei ein Kanal in eine Aufnahmeöffnung eines Ständers angeordnet wird, wobei ein reibungsarmes Gleiten in den Aufnahmeöffnungen gewährleistet ist.

Es mag einen Bedarf für eine Rohrhalterung und für einen Fermenter geben, wobei ein Rohr, insbesondere ein Heizungsrohr, mittels der Rohrhalterung zuverlässig gehaltert werden kann und wobei das Rohr Beanspruchungen (mechanischen Spannungen, Korrosion), welche innerhalb eines Biogasfermenters auftreten, standhält. Weiter mag es einen Bedarf für eine Rohrhalterung und für einen Fermenter geben, wobei die Rohrhalterung ermöglicht, insbesondere einen Heizungsrohrabschnitt innerhalb eines Fermenters derart zuverlässig zu haltern, dass ein kontinuierlicher Betrieb des Fermenters auch unter verschiedenen Temperaturbedingungen ermöglicht ist.

### Zusammenfassung der Erfindung

Gemäß einer Ausführungsform der vorliegenden Erfindung ist eine Rohrhalterung, insbesondere zum Haltern eines Heizungsrohrs oder eines Heizungsrohrabschnitts, gemäß dem Anspruch 1 bereitgestellt, welche einen Befestigungsabschnitt zum Befestigen der Rohrhalterung an einer Basisstruktur (wie etwa einer Fermenterwand) und einen Rohrhalterungsabschnitt zum Haltern eines Rohrabschnitts aufweist. Dabei ist die Rohrhalterung ausgebildet, mittels des Rohrhalterungsabschnitts den Rohrabschnitt relativ zu dem Befestigungsabschnitt verschiebbar zu haltern.

Der Befestigungsabschnitt mag einen Abschnitt der Rohrhalterung repräsentieren, welcher zur Befestigung der Rohrhalterung an einer Basisstruktur, wie etwa an einer Fermenterinnenwand, ausgebildet ist. Der Befestigungsabschnitt mag zum Beispiel eine geeignete Kontaktfläche zum Kontaktieren mit der Basisstruktur aufweisen. Weiter mag der Befestigungsabschnitt eine oder mehrere Öffnungen, wie insbesondere Bohrungen, aufweisen, durch welche eine oder mehr Schrauben oder Bolzen geführt werden können, um den Befestigungsabschnitt mit seiner Kontaktfläche in Kontakt mit der Basisstruktur, insbesondere mit einer Innenwand eines Fermenters, zu bringen und den Befestigungsabschnitt fest mit der Basisstruktur zu verbinden, insbesondere zu verschrauben. Alternativ mag der Befestigungsabschnitt in eine Öffnung der Basisstruktur einrasten oder einhaken, um die Rohrhalterung an der Basisstruktur anzubringen.

Der Rohrabschnitt mag einen kreisförmigen Querschnitt aufweisen.

Der Rohrhalterungsabschnitt mag den Rohrabschnitt aufnehmen bzw. führen, wobei der Rohrabschnitt innerhalb des Rohrhalterungsabschnitts angeordnet sein kann. Der Rohrhalterungsabschnitt mag insbesondere eine flexible bzw. lockere Halterung des Rohrabschnitts erlauben, so dass sich der Rohrabschnitt relativ zu dem Befestigungsabschnitt der Rohrhalterung innerhalb gewisser Grenzen bewegen kann. Die ermöglichte Bewegung des gehalterten Rohrabschnitts mag in verschiedenen Richtungen erfolgen, wie etwa entlang einer Längsausdehnungsrichtung des Rohrabschnitts und/oder insbesondere quer dazu. Eine ermöglichte Bewegung des gehalterten Rohrabschnitts mag während einer Temperaturänderung des Rohrabschnitts auftreten, wie sie beim Betreiben eines Biogasfermenters auftreten mag. Die durch die Rohrhalterung ermöglichte Verschiebbarkeit und/oder Beweglichkeit des gehalterten Rohrabschnitts ermöglicht oder erleichtert eine Ausdehnung des Rohrabschnitts, insbesondere bei Temperaturänderungen, wobei mögliche Spannungen aufgrund der verschiebbaren Halterung reduziert sind.

Trotz der verschiebbaren Halterung des Rohrabschnitts kann der Rohrabschnitt genügend gesichert sein. Insbesondere mag der Rohrabschnitt nur innerhalb gewisser Grenzen verschiebbar gehaltert werden. Die Grenzen der verschiebbaren Halterung können dabei abhängig von der spezifischen Anwendung gewählt werden, wie etwa abhängig von einer erwarteten Temperaturänderung, einer Länge des Rohrabschnitts, eines Materials des Rohrabschnitts, und/oder insbesondere eines Wärmeausdehnungskoeffizienten des Rohrabschnitts. Jenseits der festgelegten oder gewählten Verschiebbarkeitsgrenzen kann eine Bewegung des Rohrabschnitts verhindert bzw. unmöglich werden.

Insbesondere mag sich der Rohrabschnitt bei einer ersten Temperatur in einer ersten Gleichgewichtsausdehnung (entlang einer Längsausdehnungsrichtung des Rohrabschnitts und/oder quer dazu) befinden und während einer zweiten Temperatur in einer zweiten Gleichgewichtsausdehnung befinden (oder sich dorthin entwickeln), wobei die erste Gleichgewichtsausdehnung verschieden ist von der zweiten Gleichgewichtsausdehnung. Dabei kann sich der Rohrabschnitt bei der ersten Temperatur in einer ersten Position (relativ zu der Basisstruktur) befinden und bei der zweiten Temperatur in einer zweiten Position befinden, welche verschieden von der ersten Position ist. Insbesondere kann die zweite Position durch Verschieben der ersten Position erreicht sein. Gemäß einer Ausführungsform der Rohrhalterung ist eine solche Verschiebung von der ersten Position zu der zweiten Position ohne, bzw. nur mit einer geringfügiger mechanischer Belastung oder mechanischer Spannung des Rohrabschnitts begleitet oder verbunden. Damit kann eine mechanische Belastung des Rohrabschnitts und insbesondere eines durch eine Mehrzahl von Rohrabschnitten gebildeten Heizungsrohrs vermindert werden. Damit kann ein kontinuierlicher sicherer Betrieb eines Fermenters sichergestellt werden.

Gemäß einer Ausführungsform ist die Rohrhalterung ausgebildet, den Rohrabschnitt entlang einer ersten Richtung, welche quer, insbesondere senkrecht, zu einer Längsausdehnungsrichtung des Rohrabschnitts ausgerichtet ist, verschiebbar zu haltern.

Dabei kann eine Verschiebbarkeit des Rohrabschnitt entlang der ersten Richtung in einem ersten Verschiebbarkeitsbereich (gemessen in einer Längeneinheit) ermöglicht sein, wobei die Rohrhalterung ermöglichen kann, dass sich eine entlang der ersten Richtung gemessene Position des Rohrabschnitts innerhalb des ersten Verschiebbarkeitsbereichs ändern kann.Z. B. mag der Rohrabschnitt innerhalb einer Aussparung der Rohrhalterung entlang der ersten Richtung (welche im an einen Rund-Fermenter montierten Zustand insbesondere einer radialer Richtung des Rund-Fermenters entsprechen kann) derart verschiebbar gehaltert sein, dass sich der Rohrabschnitt entlang zumindest der ersten Richtung in gewissen Grenzen bewegen kann, z.B. in einem Verschiebbarkeitsbereich zwischen 5 cm und 40 cm, insbesondere zwischen 10 cm und 30 cm.

Wenn die Rohrhalterung an einer Innenwand eines Rund-Fermenters installiert ist, kann die erste Richtung insbesondere entlang einer radialen Richtung des Rund-Fermenters ausgerichtet sein. Insbesondere kann es abhängig von einer Temperatur des Rohrabschnitts zu verschiedenen Gleichgewichtsausdehnungen des Rohrabschnitts kommen, welche bei einem an einem Rund-Fermenter angebrachten Rohrabschnitt zu einer Veränderung eines Gesamtdurchmessers bzw. eines Gesamtradius des durch die Mehrzahl der Rohrabschnitte gebildeten Rohres führen können. Eine Verschiebbarkeit entlang der ersten Richtung, insbesondere entlang einer radialen Richtung, ermöglicht somit eine Einstellung eines Radius des aus der Mehrzahl von Rohrabschnitten gebildeten Rohres, welches innerhalb des Fermenters zum Heizen des biologischen Materials mittels der Rohrhalterung gehaltert ist, wobei mechanische Spannungen der Rohres verglichen mit einer starren Halterung (mit fixer radialer Position) vermindert sein mögen.

Gemäß einer Ausführungsform ist eine Verschiebbarkeit des Rohrabschnitt entlang einer zweiten Richtung, welche senkrecht, zu der Längsausdehnungsrichtung des Rohrabschnitts ausgerichtet ist und quer, insbesondere senkrecht, zu der ersten Richtung ausgerichtet ist, in einem zweiten Verschiebbarkeitsbereich ermöglicht, welcher zwischen 10 mal und 100 mal, insbesondere zwischen 50 mal und 1000 mal, kleiner ist als der erste Verschiebbarkeitsbereich. Insbesondere kann eine Verschiebbarkeit des Rohrabschnitt entlang der zweiten Richtung verhindert sein.

Gemäß einer Ausführungsform ist die Rohrhalterung ausgebildet, den Rohrabschnitt entlang der Längsausdehnungsrichtung des Rohrabschnitts verschiebbar zu haltern. Damit können weitere Spannungen, welche bei dem Betrieb eines Fermenters auftreten können, wie etwa Spannungen aufgrund von Temperaturänderungen, vermindert werden.

Gemäß einer Ausführungsform ist der Befestigungsabschnitt einstückig mit dem Rohrhalterungsabschnitt ausgeführt, wobei die Rohrhalterung insbesondere als ein gekantetes Blech ausgeführt ist. Insbesondere kann die Rohrhalterung als ein gekantetes Edelstahlblech ausgeführt sein, insbesondere um eine Korrosion zu vermindern. Dies mag eine besonders einfache Fertigung der Rohrhalterung ermöglichen. Insbesondere kann der Befestigungsabschnitt relativ zu dem Rohrhalterungsabschnitt abgekantet sein, wobei der abgekantete Befestigungsabschnitt eine Kontaktfläche zum Kontaktieren der Basisstruktur, insbesondere einer Fermenterinnenwand, bereitstellt. Die Rohrhalterung mag einen weiteren oder weitere abgekantete Bereiche aufweisen. Der Rohrhalterungsabschnitt mag ein Bereich in dem Blech sein, wobei das Blech in diesem Bereich des Rohrhalterungsabschnitts eine Aussparung und/oder eine besondere Kantenstruktur aufweisen mag.

Gemäß einer Ausführungsform weist die Rohrhalterung ferner eine Rohrumgreifstruktur, insbesondere einen Ring, auf, wobei die Rohrumgreifstruktur ausgebildet ist, den Rohrabschnitt (zumindest teilweise) zu umgreifen (oder zu umgeben), und wobei die Rohrumgreifstruktur in dem Rohrhalterungsabschnitt verschiebbar angeordnet werden kann.

Die Rohrumgreifstruktur mag den Rohrabschnitt ganz oder teilweise umgreifen. Dabei mag die Rohrumgreifstruktur insbesondere einen Innendurchmesser bzw. eine Innenausdehnung haben, welche gleich ist wie oder größer ist als ein Außendurchmesser bzw. eine Außenausdehnung des Rohrabschnitts. Damit kann die Rohrumgreifstruktur insbesondere den Rohrabschnitt innerhalb der Rohrumgreifstruktur aufnehmen bzw darin gleiten lassen. Insbesondere kann eine Innenausdehnung bzw. ein Innendurchmesser der Rohrumgreifstruktur um 0,5% bis 30%, insbesondere 1% bis 10%, größer sein als ein Außendurchmesser bzw. eine Außenausdehnung des Rohrabschnitts. Damit kann der Rohrabschnitt innerhalb der Rohrumgreifstruktur verschiebbar angeordnet sein bzw. gleiten.

Damit kann eine Bewegung bzw. eine Verschiebung des Rohrabschnitts bei Temperaturänderungen ermöglicht sein. Hierbei kann insbesondere eine Verschiebung des Rohrabschnitts entlang der Längsausdehnungsrichtung des Rohrabschnitts erleichtert sein. Wenn die Rohrumgreifstruktur als ein Ring ausgebildet ist, kann dieser Ring insbesondere kreisförmig sein oder zumindest eine Form einer Innenausdehnung aufweisen, welche einer Form einer Außenausdehnung des Rohrabschnitts entspricht. Insbesondere kann die Rohrhalterung ausgebildet sein, einen Rohrabschnitt mit kreisförmigem Querschnitt verschiebbar zu haltern. Ein Ring kann dabei den Rohrabschnitt vollständig umgreifen. Damit kann eine Zuverlässigkeit der Halterung verbessert werden.

Gemäß einer Ausführungsform weist die Rohrumgreifstruktur ein galvanisch isolierendes und/oder thermisch isolierendes Material, insbesondere Kunststoff, auf und ist derart anordenbar oder kann derart angeordnet werden, dass der gehalterte Rohrabschnitt von dem Rohrhalterungsabschnitt galvanisch und/oder thermisch isoliert ist.

Durch eine galvanische Trennung des Rohrabschnitts von dem Rohrhalterungsabschnitt und somit auch von dem Befestigungsabschnitt kann eine Korrosion der Rohrabschnittes und/oder der Rohrhalterung vermindert werden. Weiterhin kann eine Wärmeabfuhr von dem Rohrabschnitt zu dem Rohrhalterungsabschnitt und auch zu dem Befestigungsabschnitt vermindert werden. Damit ist insbesondere eine Wärmeabfuhr von dem Rohrabschnitt zu der festen Basisstruktur, insbesondere einer Fermenterinnenwand, vermindert. Insbesondere mag die Rohrumgreifstruktur als ein elektrisch/galvanisch/thermisch wirkendes Isolationselement ausgeführt sein. Der Ring bzw. die Rohrumgreifstruktur kann insbesondere aus Polypropylen (PP), Polyoxymethylen (POM), einem beliebigen thermoplastischen und/oder thermohärtenden Kunststoff, einem beliebigen anderen Kunststoff und/oder einem sonstigen isolierenden Material gefertigt sein oder diese Materialien aufweisen.

Gemäß einer Ausführungsform der vorliegenden Erfindung weist der Rohrhalterungsabschnitt zwei gerade, insbesondere parallele, Kanten auf, welche entlang der ersten Richtung ausgerichtet sind, wobei zwischen den zwei Kanten die Rohrumgreifstruktur derart aufgenommen werden kann, dass die Rohrumgreifstruktur entlang der zwei geraden Kanten gleiten kann. Insbesondere können die zwei geraden Kanten in dem gekanteten Blech ausgeführt sein. Der Rohrabschnitt kann auch ohne eine Rohrumgreifstruktur entlang der zwei parallelen Kanten gleiten, die Rohrhalterung muss also nicht notwendigerweise die Rohrumgreifstruktur aufweisen.

Die Rohrumgreifstruktur kann derart ausgeführt sein, dass eine erste Punktberührung zu einer ersten der zwei Kanten und eine zweite Punktberührung zu einer zweiten der zwei geraden Kanten erfolgt. Eine Punktberührung mag eine Verkantung der Rohrumgreifstruktur vermindern, um eine Verschiebbarkeit des gehalterten Rohrabschnitts zu erleichtern. Während einer Verschiebung des Rohrabschnitts mag die Rohrumgreifstruktur zumindest teilweise eine Rollbewegung ausführen. Dabei können mögliche Kontaktpunkte der Rohrumgreifstruktur mit der ersten der zwei geraden Kanten und der zweiten der zwei geraden Kanten auf einem Kreis liegen. Alternativ können die Kontaktpunkte auch auf einer ovalen Form liegen. Die zwei parallelen geraden Kanten können in einer gemeinsamen Ebene senkrecht zu der Längsausdehnungsrichtung des Rohrabschnitts liegen. Die Kanten des Rohrhalterungsabschnitts können Kanten eines Edelstahlblechs sein. Die Kanten können mit einem Isolationsüberzug (etwa aus Kunststoff) zur thermischen und/oder galvanischen Isolation versehen sein.

Gemäß einer Ausführungsform der Erfindung sind die zwei geraden Kanten über einen gebogenen (insbesondere halbkreisförmigen) Kantenabschnitt verbunden, wobei der gebogene Kantenabschnitt näher bei dem Befestigungsabschnitt angeordnet ist als die zwei geraden Kanten.

Insbesondere kann der gebogene Kantenabschnitt eine Begrenzung der Verschiebbarkeit des Rohrabschnitts entlang der ersten Richtung definieren, mithin den ersten Verschiebbarkeitsbereich teilweise festlegen. Insbesondere kann der maximal verschobene Rohrabschnitt (entlang der ersten Richtung) an dem gebogenen Kantenabschnitt anstoßen, um eine weitere Bewegung entlang der ersten Richtung zu verhindern. Der gebogene Kantenabschnitt kann dabei in einer gemeinsamen Ebene mit der gemeinsamen Ebene der zwei geraden Kanten liegen. Dabei kann der gebogene Kantenabschnitt insbesondere ein Kantenabschnitt eines Edelstahlblechs sein. In einem montierten Zustand kann der gebogene Kantenabschnitt näher an der Fermenterinnenwand liegen als die zwei geraden Kanten, um so eine Montage des Rohrabschnitts von einem Innenraum her zu erleichtern.

Gemäß einer Ausführungsform der Erfindung sind die zwei geraden Kanten mittels einer Aussparung in der Rohrhalterung gebildet, wobei der Rohrabschnitt innerhalb der Aussparung halterbar ist. Insbesondere kann die Aussparung lediglich durch die zwei geraden Kanten und den gebogenen Kantenabschnitt begrenzt sein, während die Aussparung in einem dem gebogenen Kantenabschnitt gegenüberliegenden Bereich keine Begrenzung aufweisen mag. Über diesen dem gebogenen Kantenabschnitt gegenüberliegenden Bereich kann somit der Rohrabschnitt zwischen die zwei geraden Kanten innerhalb der Aussparung angeordnet werden. Damit kann eine Montage des Rohrabschnitts erleichtert werden.

Gemäß einer Ausführungsform der Erfindung weist die Rohrhalterung ferner ein Verschlusselement zum Verschließen der Aussparung auf, wobei das Verschlusselement insbesondere zum Verschließen der Aussparung in einem dem gebogenen Kantenabschnitt gegenüberliegenden Bereich ausgebildet ist. Damit kann nach Anordnen des Rohrabschnitts zwischen den zwei geraden Kanten innerhalb der Aussparung der Aussparungsbereich, welcher zum Verschieben des Rohrabschnitts verwendet werden kann, durch das Verschlusselement begrenzt werden, um mithin der ersten Verschiebbarkeitsbereich weiter zu definieren.

Das Verschlusselement mag insbesondere als eine Abschlussleiste ausgeführt sein. Insbesondere mag dazu das Blech der Rohrhalterung einen weiteren abgekanteten bzw. gekanteten Bereich aufweisen, welcher relativ zum Rohrhalterungsabschnitt um insbesondere 90° gekantet ist, wobei an diesem abgekanteten Bereich das Verschlusselement (beispielsweise durch einen Bolzen oder eine Schraube) angebracht werden kann. Damit kann eine Verschiebbarkeit des Rohrabschnitts innerhalb der Aussparung begrenzt werden, um den Rohrabschnitt innerhalb eines definierten Verschiebbarkeitsbereiches verschiebbar oder beweglich anzuordnen.

Gemäß einer Ausführungsform der Erfindung weist die Rohrumgreifstruktur ein Querschnittsprofil (insbesondere ein Profil entlang der Längsausdehnungsrichtung des umgriffenen Rohrabschnitts) mit einer, insbesondere kreisförmigen, Nut auf, wobei die zwei geraden Kanten die Nut derart gleitfähig aufnehmen, dass die Rohrumgreifstruktur entlang der ersten Richtung entlang der zwei geraden Kanten gleiten kann und derart, dass die Rohrumgreifstruktur an einer Bewegung entlang einer von der ersten Richtung verschiedenen (insbesondere der zweiten) Richtung gehindert ist.

Die Rohrumgreifstruktur mag ein Querschnittsprofil mit einer oberen Außenausdehnung, einer mittleren Außenausdehnung und einer unteren Außenausdehnung haben, wobei die obere und untere Außenausdehnung beide größer sind als die mittlere Außenausdehnung. Die mittlere Außenausdehnung mag bemessen sein, um zwischen die zwei geraden Kanten zu passen. Die obere Außenausdehnung und die untere Außenausdehnung mögen derart gewählt sein, dass sie größer als der Abstand der zwei geraden Kanten sind. Damit kann eine gleitfähige Führung der Rohrumgreifstruktur entlang der zwei geraden Kanten ermöglicht werden, wobei die Rohrumgreifstruktur an einer Bewegung senkrecht zu einer durch die zwei geraden Kanten aufgespannten Ebene gehindert sein mag. Dabei kann die Nut dem Bereich der mittleren Außenausdehnung der Rohrumgreifstruktur entsprechen. Insbesondere mag die Rohrumgreifstruktur ein Querschnittsprofil einer U-Form aufweisen.

Gemäß einer Ausführungsform der Rohrhalterung ist eine Fläche des Befestigungsabschnitts relativ zu einer Fläche des Rohrhalterungsabschnitts gekantet, insbesondere um 90°. Dabei kann insbesondere der Rohrhalterungsabschnitt senkrecht zu einer Basisstruktur, insbesondere einer Fermenterinnenwand, ausgerichtet sein, falls die Rohrhalterung an der Basisstruktur befestigt ist.

Gemäß einer Ausführungsform der Erfindung ist die Rohrhalterung zum Haltern von vier Rohrabschnitten ausgebildet, welche entlang einer zweiten Richtung voneinander beabstandet sind, wobei die zweite Richtung quer, insbesondere senkrecht, zu der ersten Richtung und quer, insbesondere senkrecht, zu der Längsausdehnungsrichtung des jeweiligen Rohrabschnitts ausgerichtet ist. In einem an einer Innenwand eines Fermenters installierten Zustand können die vier Rohrabschnitte vertikal übereinander angeordnet sein. Damit kann die Rohrhalterung vorteilhaft zum Haltern einer Vielzahl von Rohren eingesetzt werden. Gemäß alternativen Ausführungsformen der Erfindung ist die Rohrhalterung ausgebildet, zwei, drei, fünf, sechs, sieben oder acht bis zehn Rohrabschnitte zu haltern.

Gemäß einer Ausführungsform ist ein Fermenter (insbesondere Rund-Fermenter zum Erzeugen von Biogas) zum Fermentieren von biologischem Material in einem Innenraum des Fermenters bereitgestellt, wobei der Fermenter eine Fermenterwand, insbesondere mit kreisförmigem Querschnitt, zum Begrenzen des Innenraums; eine Rohrhalterung nach einer der oben beschriebenen Ausführungsformen, welche mittels des Befestigungsabschnitts der Rohrhalterung an einer Innenseite der Fermenterwand angebracht ist; und mindestens einen Rohrabschnitt aufweist, welcher mittels des Rohrhalterungsabschnitts der Rohrhalterung gehaltert ist.

Dabei ist der Rohrabschnitt zum Heizen des Innenraums des Fermenters angeordnet und die erste Richtung ist entlang einer horizontalen Richtung, insbesondere einer radialen Richtung des Fermenters, ausgerichtet. Der Fermenter kann als eine Biogasanlage zum Erzeugen von Methan durch einen mikrobiellen Abbauprozess von organischen Substanzen bzw. biologischem Material ausgebildet sein. Die Fermenterwand mag insbesondere aus Beton gefertigt sein. In dem Innenraum des Fermenters kann das organische Material bzw. das biologische Material angeordnet sein und bewegt werden. Dabei können aggressive, insbesondere saure Bedingungen herrschen, welche Komponenten des Fermenters chemisch angreifen können.

Der Rohrabschnitt mag ein Abschnitt eines Heizungsrohrs sein, wobei das Heizungsrohr das biologische Material in dem Innenraum des Fermenters heizt. Dabei können ein oder mehrere Heizrohre oder Heizleitungen in einer oder mehreren horizontalen Ebenen vertikal übereinander angeordnet sein. Dabei ist der Rohrabschnitt derart durch die Rohrhalterung verschiebbar gehaltert, dass sich der Rohrabschnitt, insbesondere bei Temperaturänderungen, entlang einer radialen Richtung zum Ändern eines Radius bzw. eines Durchmessers des Heizungsrohrs, verschieben kann. Damit können Spannungen des Heizungsrohrs, welche insbesondere bei Temperaturänderung auftreten, vermindert werden. Nichtsdestotrotz kann das Heizungsrohr zuverlässig und sicher an der Fermenterwand gehaltert werden.

Gemäß einer Ausführungsform lastet auf einer der zwei geraden Kanten ein Gewicht des Rohrabschnitts, insbesondere vermittelt über eine Rohrumgreifstruktur, welche zwischen den zwei geraden Kanten gleitfähig aufgenommen ist.

Weiterhin mag der Fermenter ein Heizungssystem aufweisen, welches das eine oder die mehr Heizungsrohre mit Heizflüssigkeit einer geeigneten Temperatur versorgt. Dazu können das eine bzw. die mehr Heizungsrohre durch die Fermenterwand nach außen geführt werden, um die Heizungsflüssigkeit zu- und auch wieder abzuführen.

Die Heizungsrohre können aus einer Mehrzahl von Rohrabschnitten zusammengesetzt sein, wobei jeder Rohrabschnitt eine Länge zwischen etwa 1 m und 10 m, insbesondere zwischen 3 m und 7 m, aufweist. Insbesondere können zwischen 10 und 15 Rohrabschnitte miteinander verschweißt werden, um ein Heizungsrohr zu bilden. Ein Durchmesser des Fermenters mag zwischen 20 m und 30 m liegen. Insbesondere mag der Fermenter vier vertikal übereinander angeordnete Heizungsrohre aufweisen, welche durch eine Mehrzahl von Rohrhalterungen gehaltert sind. Insbesondere können drei Rohrhalterungen zum Haltern eines Rohrabschnitts einer Länge von etwa 6 m verwendet werden.

Die Rohrhalterung mag eine Ausdehnung entlang der ersten Richtung zwischen 20 cm und 60 cm, insbesondere zwischen 30 cm und 50 cm, aufweisen und/oder eine Ausdehnung entlang der zweiten Richtung zwischen 50 cm und 200 cm, insbesondere zwischen 70 cm und 130 cm, aufweisen. Eine Länger der zwei geraden Kanten mag zwischen 10 cm und 30 cm, insbesondere zwischen 15 cm und 25 cm, liegen, wobei die zwei Kanten einen Abstand zwischen 5 cm und 20 cm, insbesondere zwischen 7 cm und 13 cm, aufweisen können. Damit mag mittels der Rohrhalterung ein Rohrabschnitt eines Durchmessers zwischen 3 cm und 10 cm, insbesondere zwischen 4 cm und 7 cm, verschiebbar gehaltert sein.

### Kurze Beschreibung der Zeichnungen

Fig. 1A illustriert eine Draufsicht einer Rohrhalterung gemäß einer Ausführungsform der Erfindung;
Fig. 1B illustriert eine Seitenansicht der in Fig. 1A illustrierten Rohrhalterung;
Fig. 1C illustriert eine Vorderansicht der in Fig. 1A illustrierten Rohrhalterung;
Fig. 2 illustriert perspektivische Ansichten der in Fig. 1A illustrierten Rohrhalterung;
Fig. 3A illustriert eine Draufsicht einer Rohrumgreifstruktur, welche in der Rohrhalterung gemäß Fig. 1A eingesetzt wird;
Fig. 3B illustriert eine Querschnittsansicht der in Fig. 3A illustrierten Rohrumgreifstruktur;
Fig. 4 illustriert ein Verschlusselement, welches in der in Fig. 1A illustrierten Rohrhalterung eingesetzt wird; und
Fig. 5 illustriert eine Querschnittsansicht eines Fermenters gemäß einer Ausführungsform der Erfindung.

Nachfolgend werden Ausführungsformen der vorliegenden Erfindung anhand der beiliegenden Zeichnungen erläutert. Die Erfindung ist nicht auf die illustrierten oder beschriebenen Ausführungsformen beschränkt.

### Detaillierte Beschreibung von einigen Ausführungsformen

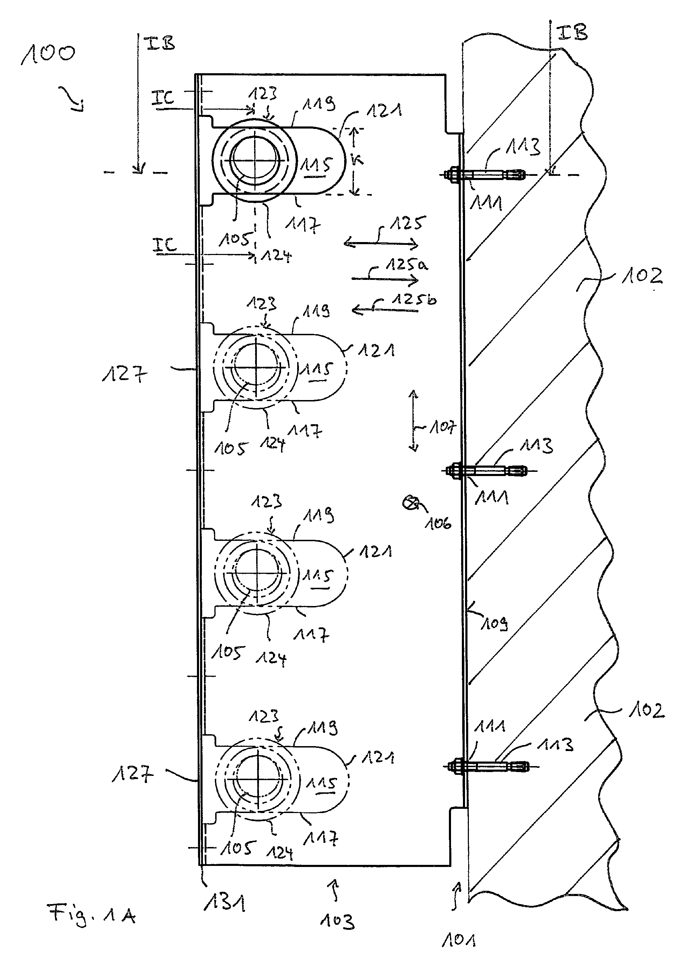

Fig. 1A illustriert eine Draufsicht einer Rohrhalterung 100 gemäß einer Ausführungsform der Erfindung. Die Rohrhalterung 100 weist einen Befestigungsabschnitt 101 zum Befestigen der Rohrhalterung an einer Basisstruktur 102, wie etwa an einer Fermenterinnenwand, auf und einen Rohrhalterungsabschnitt 103 zum Haltern eines Rohrabschnitts 105 (mit z.B. kreisförmigem Querschnitt).

Insbesondere ist die in Fig. 1A in einer Draufsicht illustrierte Rohrhalterung 100 zum Haltern von vier Rohrabschnitten 105 ausgebildet, welche entlang einer zweiten Richtung 107 voneinander beabstandet sind. In einem an einer Fermenterinnenwand montierten Zustand verläuft die zweite Richtung 107 in einer vertikalen Richtung.

Der Befestigungsabschnitt 101 und der Rohrhalterungsabschnitt 103 sind einstückig durch ein gekantetes Blech, insbesondere ein Edelstahlblech, gebildet. Dabei liegt eine Fläche des Rohrhalterungsabschnitts in der Zeichenebene der Fig. 1A und eine Fläche des Befestigungsabschnitts 101 (und eine Fläche der Fermenterinnenwand 102) liegt senkrecht zu der Zeichnungsebene der Fig. 1A.

Der Befestigungsabschnitt 101 weist eine Kontaktfläche 109 auf, welche mit der Innenwand 102 des Fermenters in Kontakt gebracht werden kann. Zum Befestigen des Befestigungsabschnitts 101 an der Fermenterwand 102 weist der Befestigungsabschnitt 101 drei Öffnungen oder Bohrungen 111 auf, durch welche jeweils eine Schraube 113 geführt wird, welche den Befestigungsabschnitt 101 mit der Fermenterinnenwand 102 verbindet.

Der Rohrhalterungsabschnitt 103 weist für jeden Rohrabschnitt 105 jeweils eine Aussparung 115 auf, welche durch zwei gerade Kanten 117, 119 und eine gebogene Kante 121 gebildet sind. Zwischen den zwei geraden Kanten 117, 119 ist ein Ring bzw. eine Rohrumgreifstruktur 123 angeordnet, welche den Rohrabschnitt 105 vollständig umgreift, sodass der Rohrabschnitt 105 zwischen den zwei geraden Kanten 117, 119 verschiebbar gehaltert ist. Die Rohrumgreifstruktur 123 kann entlang der geraden Kanten 117, 119 entlang einer ersten Richtung, welche durch Doppelpfeil 125 gekennzeichnet ist, gleiten, so dass der in dem Ring 123 aufgenommene Rohrabschnitt 105 entlang der ersten Richtung 125 verschiebbar durch die Rohrhalterung 100 gehaltert ist.

Dabei begrenzt die gebogene Kante 121 die Bewegung bzw. die Verschiebbarkeit des Rohrabschnitts 105 entlang der positiven ersten Richtung 125a und eine Abschlussleiste bzw. ein Verschlusselement 127 begrenzt eine Verschiebbarkeit des Rohrabschnitts 105 entlang der negativen ersten Richtung 125b in einer Gegenrichtung.

Im Sinne der vorliegenden Anmeldung wird eine Bewegung/Verschiebbarkeit entlang der ersten Richtung 125 als eine Bewegung in positiver erster Richtung 125a oder negativer erster Richtung 125b verstanden. Insbesondere wird eine positive Richtung der ersten Richtung 125 als eine Richtung 125a und eine negative Richtung der ersten Richtung 125 als eine Richtung 125b bezeichnet. Dabei begrenzt insbesondere die gebogene Kante 121 eine Bewegung oder eine Verschiebbarkeit des Rohrabschnitts 105 in der positiven ersten Richtung 125a und die Abschlussleiste 127 begrenzt eine Bewegung bzw. Verschiebbarkeit des Rohrabschnitts 125 entlang der negativen ersten Richtung 125b. Gemäß der in Fig. 1A in Draufsicht illustrierten Ausführungsform der Rohrhalterung 100 ist eine Verschiebbarkeit entlang der zweiten Richtung 107 behindert bzw. nicht ermöglicht. Weiterhin ermöglicht jedoch die in Fig. 1a illustrierte Rohrhalterung 100 eine Verschiebbarkeit des Rohrabschnitts 105 entlang einer Richtung 106, welche senkrecht auf der Zeichenebene der Fig. 1A steht und insbesondere einer Längsausdehnungsrichtung des Rohrabschnitts 105 entspricht.

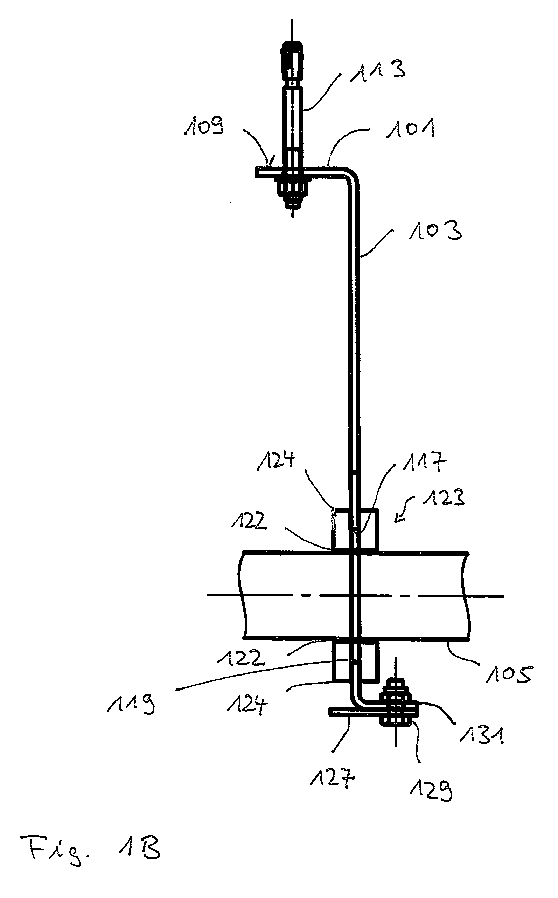

Fig. 1B illustriert eine Seitenansicht der in Fig. 1A illustrierten Rohrhalterung 100 entlang der Richtung, welche in Fig. 1A durch IB gekennzeichnet ist. Wie in Fig. 1B illustriert, ist die Abschlussleiste bzw. das Verschlusselement 127 mittels einer oder mehrerer Bolzen oder Schrauben 129 an dem

Rohrhalterungsabschnitt 103, insbesondere an einem abgekanteten Abschnitt 131 der Rohrhalterung angebracht, um die Aussparung 115 entlang der negativen ersten Richtung 125b zu begrenzen.

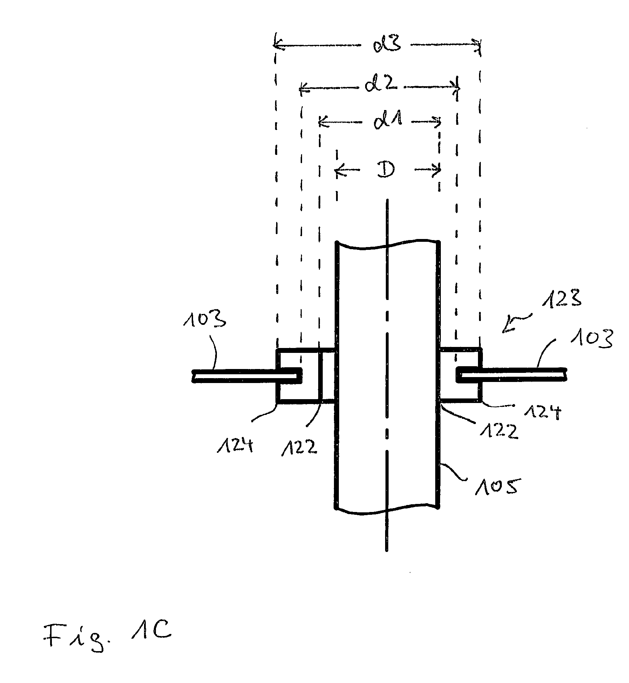

Fig. 1C illustriert eine Vorderansicht der in Fig. 1A illustrierten Rohrhalterung 100, wobei die Vorderansicht entlang der in Fig. 1A mit IC gekennzeichneten Richtung genommen ist. Wie aus insbesondere Fig. 1C ersichtlich, ist ein Innendurchmesser d1 des Ringes 123 kleiner als ein Außendurchmesser D des Rohrabschnitts 105. Damit kann der Rohrabschnitt 105 innerhalb des Ringes 123 gleiten und ist somit verschiebbar. Weiter weist der Ring 123 in einem mittleren Bereich im Querschnitt eine Nut auf, welche ein Führen des Ringes 123 zwischen den geraden Kanten 117 und 119 ermöglicht.

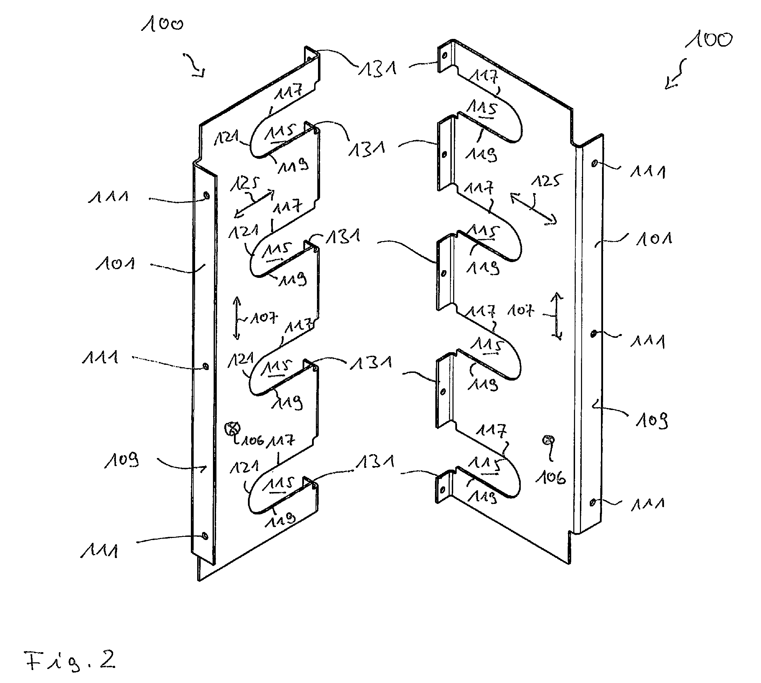

Fig. 2 zeigt zwei 90° relativ zueinander verdrehte perspektivische Ansichten der in Fig. 1A illustrierten Rohrhalterung 100, wobei jedoch die Ringe 123 und das Verschlusselement 127 nicht illustriert sind. Wie aus Fig. 2 ersichtlich ist, ist die Rohrhalterung 100 aus einem gekanteten Blech gebildet, wobei der Befestigungsabschnitt 101 relativ zu dem Rohrhalterungsabschnitt 103 um 90° abgekantet ist und wobei ein Verschlusselementbereich 131 ebenfalls relativ zu dem Rohrhalterungsabschnitt 103 um 90° abgekantet ist. In dem durch das Verschlusselement 127 nicht abgeschlossenen Zustand ist die Aussparung 115 zu der Seite des Verschlusselementbereichs 131 hin geöffnet, um somit in einfacher Weise einen Rohrabschnitt 105 zwischen den Kanten 117 und 119 anzuordnen zu können, wobei der Rohrabschnitt 105 vorzugsweise von einem Ring 123 umgeben ist und die Nut des Ringes zwischen den Kanten 117, 119 eingeführt wird.

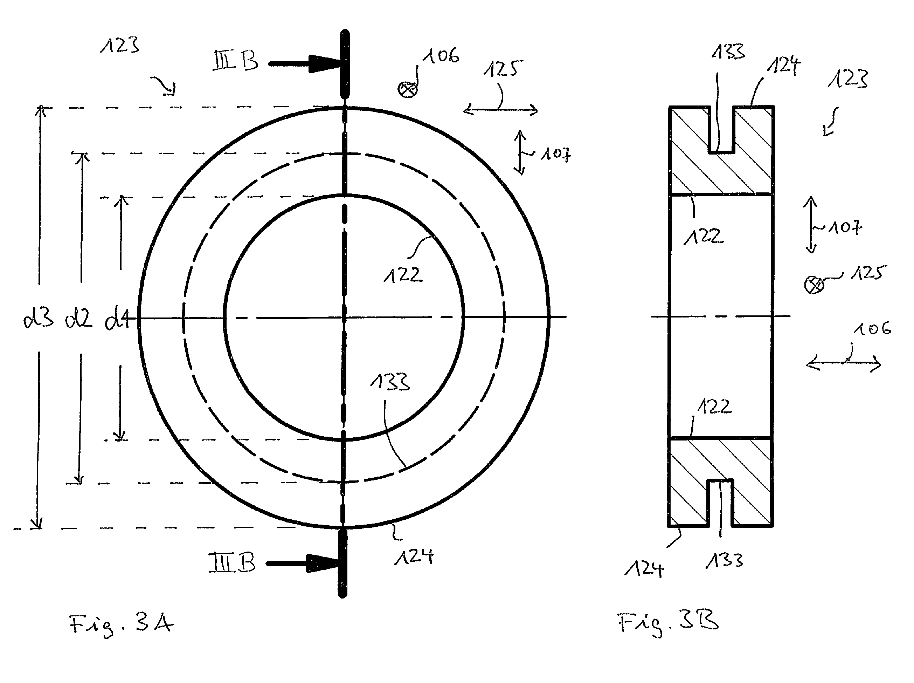

Fig. 3A zeigt eine Draufsicht und Fig. 3B zeigt eine Querschnittsansicht eines Ringes 123, welcher in der in Fig. 1A illustrierten Rohrhalterung eingesetzt werden kann. Der Ring 123 weist einen Innendurchmesser d1 einer Innenbereiches 122 auf, einen Nutdurchmesser d2 der Nut 133 auf und einen Außendurchmesser d3 eines Außenbereichs 124 auf. Insbesondere ist der Innendurchmesser d1 kleiner als ein Außendurchmesser D des Rohrabschnitts 105, damit sich der Rohrabschnitt 105 innerhalb des Ringes (insbesondere innerhalb des freien Innenbereiches 122) entlang seiner Längsausdehnungsrichtung 106 bewegen kann. Der Nutdurchmesser d2 ist derart bemessen, dass die Nut zwischen den beiden Kanten 117, 119, welche einen Abstand K voneinander beabstandet sind, angeordnet werden kann und zwischen den beiden Kanten 117, 119 verschoben werden kann. Insbesondere berührt die Nut 133 des Ringes 123 eine oder beide der geraden Kanten 117, 119 in einer Punktberührung.

Der Ring weist einen Außendurchmesser d3 auf, welcher größer ist als der Abstand K der zwei geraden Kanten 117, 119, so dass der Ring zwar entlang der ersten Richtung 125 verschoben werden kann, jedoch nicht entlang weder der Richtung 106 einer Längsausdehnungsrichtung des Rohrabschnitts noch entlang der zweiten Richtung 107. Der Ring 123 ist aus Kunststoff gefertigt, insbesondere aus PP oder POM. Damit kann der Ring 123 gewährleisten, dass der Rohrabschnitt 105 nicht in galvanischen und/oder thermischen Kontakt mit dem Rohrhalterungsabschnitt 103 und somit auch nicht mit der Fermenterwand 102 kommt.

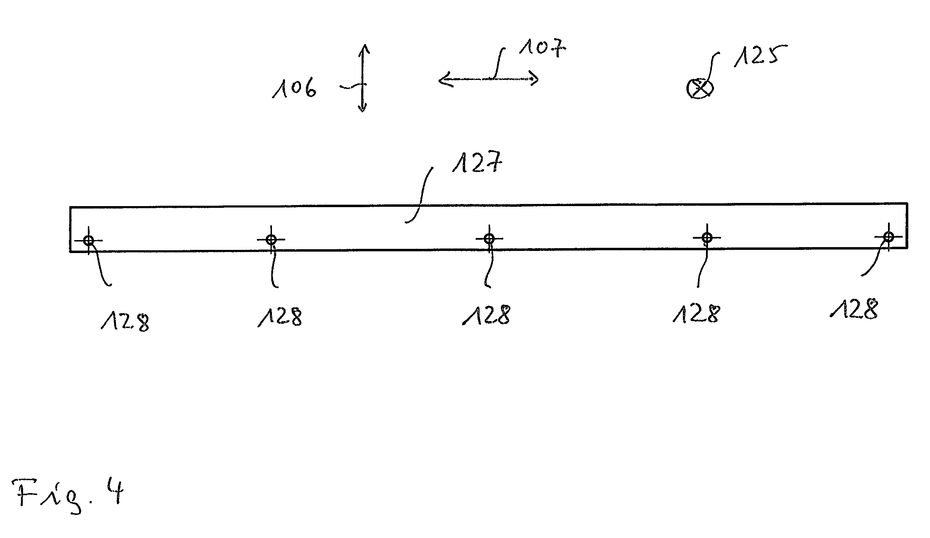

Fig. 4 illustriert eine Abschlussleiste bzw. ein Verschlusselement 127, welches in der in Fig. 1A illustrierten Rohrhalterung 100 eingesetzt werden kann. Das Verschlusselement 127 ist als ein Blechstreifen ausgebildet, welches fünf Öffnungen bzw. Bohrungen 128 aufweist, mit Hilfe derer das Verschlusselement 127 an den Verschlusselementbereich 131 der Rohrhalterung 100 angebracht werden kann.

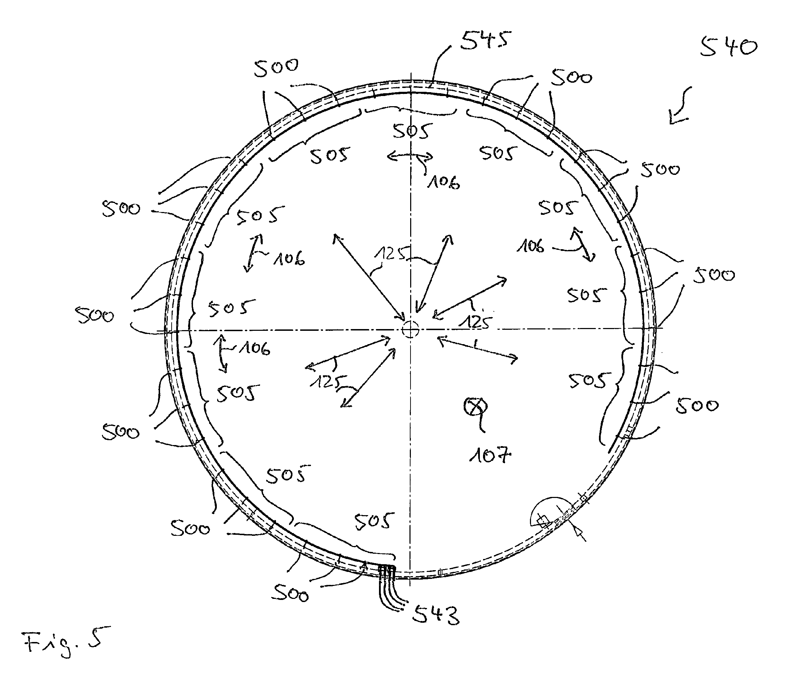

Fig. 5 illustriert in schematischer Weise eine Querschnittsansicht eines Fermenters 540 gemäß einer Ausführungsform der Erfindung. Bei dem Fermenter 540 handelt es sich um einen Rund-Fermenter, welcher eine kreisförmige Querschnittsform hat. Der Fermenter 540 kann zur Fermentierung, das heißt mikrobiellen Vergärung von biologischem oder organischem Material verwendet werden, wobei zur Unterstützung des mikrobiellen Abbauprozesses ein Innenraum 541 des Fermenters 540 durch vier Heizungsrohre 543 geheizt wird. Dabei ist jedes der Heizungsrohre 543 durch elf Rohrabschnitte 505 gebildet.

Der Innenraum 541 des Fermenters 540 ist durch eine Fermenterwand 545 begrenzt, an welcher eine Mehrzahl von Rohrhalterungen 500 mittels jeweils des Befestigungsabschnitts 101 befestigt sind, welche jeweils vier Rohrabschnitte 505 verschiebbar haltern. Insbesondere erlauben die Rohrhalterungen 500 eine Verschiebbarkeit der Rohrabschnitte 505 entlang einer radialen Richtung 125, um Längenänderungen der Rohrabschnitte 505 bzw. der Heizungsrohre 543 während eines Betriebes des Fermenters 540 zu erlauben. Bei Ausdehnung der Rohrabschnitte 505 vergrößert sich dabei insbesondere ein Durchmesser oder ein Radius der Heizungsrohre 543, um daraufhin zu einer Verschiebung der Rohrabschnitte 505 entlang der radialen Richtung 125 nach außen zu führen. Damit können insbesondere mechanische Belastungen oder Spannungen innerhalb der Rohrabschnitte 505 bzw. der Heizungsrohre 543 beim Betrieb des Fermenters 540 verringert werden.

Die Heizungsrohre 543 können weiterhin an eine Heizungsanlage gekoppelt sein, welche den Heizungsrohren 543 Heizflüssigkeit zu- oder auch abführen kann. Wie aus Fig. 5 ersichtlich ist, werden jeweils drei Rohrhalterungen 500 zum verschiebbaren Haltern von vier Rohrabschnitten 505 jeweils einer Länge von 6 m verwendet. In anderen Ausführungsformen können mehr oder weniger Rohrhalterungen zur Halterung eines Rohrabschnitts verwendet werden und es können mehr oder weniger als vier Heizungsrohre vertikal untereinander innerhalb des Fermenters 540 angeordnet und durch ein- und dieselbe Rohrhalterung 500 gehaltert sein.

## Patentansprüche

1. Rohrhalterung (100, 500), aufweisend:
einen Befestigungsabschnitt (101) zum Befestigen der Rohrhalterung an einer Basisstruktur (102);
einen Rohrhalterungsabschnitt (103) zum Haltern eines Rohrabschnitts (105, 505);
wobei die Rohrhalterung ausgebildet ist, mittels des Rohrhalterungsabschnitts den Rohrabschnitt relativ zu dem Befestigungsabschnitt verschiebbar zu haltern, wobei die Rohrhalterung ausgebildet ist, den Rohrabschnitt entlang einer ersten Richtung (125, 125a, 125b), welche quer zu einer Längsausdehnungsrichtung (106) des Rohrabschnitts (105) ausgerichtet ist, verschiebbar zu haltern,
wobei die Rohrhalterung ferner
eine Rohrumgreifstruktur (123)
aufweist, wobei die Rohrumgreifstruktur ausgebildet ist, den Rohrabschnitt zu umgreifen und in dem Rohrhalterungsabschnitt (103) verschiebbar angeordnet werden zu können,
wobei der Rohrhalterungsabschnitt (103) zwei gerade Kanten (117, 119) aufweist, welche entlang der ersten Richtung (125) ausgerichtet sind, wobei zwischen den zwei Kanten die Rohrumgreifstruktur (123) derart aufgenommen werden kann, dass die Rohrumgreifstruktur entlang der zwei geraden Kanten (117, 119) gleiten kann,
wobei die Rohrumgreifstruktur (123) ein Querschnittsprofil mit einer Nut (133) aufweist, wobei die zwei geraden Kanten (117, 119) die Nut (133) derart gleitfähig aufnehmen, dass die Rohrumgreifstruktur (123) entlang der ersten Richtung (125) entlang der zwei geraden Kanten (117, 119) gleiten kann und dass die Rohrumgreifstruktur (123) an einer Bewegung entlang einer von der ersten Richtung verschiedenen Richtung gehindert ist,
wobei die Rohrumgreifstruktur (123) als ein Ring ausgebildet ist, der in einem mittleren Bereich im Querschnitt die Nut (133) aufweist, die sowohl um den Ring umläuft als auch um eine Längsachse des Rohrabschnitts umläuft, wenn der Rohrabschnitt in dem Ring aufgenommenen ist.

2. Rohrhalterung gemäß Anspruch 1, wobei die erste Richtung (125, 125a, 125b) senkrecht zu einer Längsausdehnungsrichtung (106) des Rohrabschnitts (105) ausgerichtet ist.

3. Rohrhalterung gemäß Anspruch 1 oder 2, welche ausgebildet ist, den Rohrabschnitt entlang der Längsausdehnungsrichtung (106) des Rohrabschnitts (105) verschiebbar zu haltern.

4. Rohrhalterung gemäß einem der Ansprüche 1 bis 3, wobei der Befestigungsabschnitt (101) einstückig mit dem Rohrhalterungsabschnitt (103) ausgeführt ist, wobei die Rohrhalterung insbesondere als ein gekantetes Blech ausgeführt ist.

5. Rohrhalterung gemäß einem der Ansprüche 1 bis 4, wobei die Rohrumgreifstruktur (123) ein galvanisch isolierendes und/oder thermisch isolierendes Material, insbesondere Kunststoff, aufweist und derart anordenbar ist, dass der gehalterte Rohrabschnitt (105) von dem Rohrhalterungsabschnitt (103) galvanisch und/oder thermisch isoliert ist.

6. Rohrhalterung gemäß einem der Ansprüche 1 bis 5, wobei die zwei gerade Kanten (117, 119) parallel sind.

7. Rohrhalterung gemäß Anspruch 6, wobei die zwei geraden Kanten (117, 119) über einen gebogenen Kantenabschnitt (121) verbunden sind, welcher näher bei dem Befestigungsabschnitt (101) angeordnet ist als die zwei geraden Kanten (117, 119).

8. Rohrhalterung gemäß Anspruch 6 oder 7, wobei die zwei geraden Kanten mittels einer Aussparung (115) in der Rohrhalterung gebildet sind, wobei der Rohrabschnitt (105) innerhalb der Aussparung (115) halterbar ist.

9. Rohrhalterung gemäß Anspruch 8, ferner aufweisend ein Verschlusselement (127) zum Verschließen der Aussparung.

10. Rohrhalterung gemäß einem der Ansprüche 1 bis 9, wobei die Nut (133) kreisförmig ist..

11. Rohrhalterung gemäß einem der vorangehenden Ansprüche, wobei eine Fläche des Befestigungsabschnitts (101) relativ zu einer Fläche des Rohrhalterungsabschnitts (103) gekantet ist, insbesondere um 90°.

12. Rohrhalterung gemäß einem der vorangehenden Ansprüche, welcher zum Haltern von vier Rohrabschnitten (105) ausgebildet ist, welche entlang einer zweiten Richtung (107) voneinander beabstandet sind, wobei die zweite Richtung quer, insbesondere senkrecht, zu der ersten Richtung (125) und quer, insbesondere senkrecht, zu der Längsausdehnungsrichtung (106) des jeweiligen Rohrabschnitts (105) ausgerichtet ist.

13. Fermenter (540) zum Fermentieren von biologischem Material in einem Innenraum (541) des Fermenters, aufweisend:
eine Fermenterwand (545), insbesondere mit kreisförmigem Querschnitt, zum Begrenzen des Innenraums (541);
eine Rohrhalterung (500) nach einem der Ansprüche 1 bis 12, welche mittels des Befestigungsabschnitts der Rohrhalterung an einer Innenseite der Fermenterwand (545) angebracht ist; und
mindestens einen Rohrabschnitt (505), welcher mittels des Rohrhalterungsabschnitts der Rohrhalterung gehaltert ist,
wobei der Rohrabschnitt zum Heizen des Innenraums (541) des Fermenters angeordnet ist und wobei die erste Richtung (125) entlang einer horizontalen Richtung, insbesondere einer radialen Richtung des Fermenters, ausgerichtet ist.

14. Fermenter nach Anspruch 13, wobei auf einer der zwei geraden Kanten ein Gewicht des Rohrabschnitts lastet, vermittelt über die Rohrumgreifstruktur, welche zwischen den zwei geraden Kanten gleitfähig aufgenommen ist.

## Claims

1. Pipe holder (100, 500), comprising:
a fixing section (101) for fixing the pipe holder to a basis structure (102);
a pipe holder section (103) for holding a pipe section (105, 505);
wherein the pipe holder is formed to slidably hold the pipe section in relation to the fixing section by the pipe holder section,
wherein the pipe holder is formed to hold the pipe section slidably along a first direction (125, 125a, 125b) which is aligned transverse to a longitudinal extension direction (106) of the pipe section (105),
wherein the pipe holder further comprises
a pipe encompassing structure (123), wherein the pipe encompassing structure is formed to encompass the pipe section and to be slidably arrangeable in the pipe holder section (103),
wherein the pipe holder section (103) comprises two straight edges (117, 119) which are aligned along the first direction (125), wherein between the two edges, the pipe encompassing structure (123) can be accommodated in such a way that the pipe encompassing structure can slide along the two straight edges (117, 119),
wherein the pipe encompassing structure (123) comprises a cross-sectional profile with a groove (133), wherein the two straight edges (117, 119) slidably accommodate the groove (133) in such a way that the pipe encompassing structure (123) can slide along the first direction (125) along the two straight edges (117, 119) and that the pipe encompassing structure (123) is prevented from a move along a direction different to the first direction,
wherein the pipe encompassing structure (123) is formed as a ring which comprises the groove (133) in a central area in the cross-section, which encircles both the ring and a longitudinal axis of the pipe section, when the pipe section is accommodated in the ring.

2. Pipe holder according to claim 1, wherein the first direction (125, 125a, 125b) is aligned perpendicular to a longitudinal extension direction (106) of the pipe section (105).

3. Pipe holder according to claim 1 or 2, which is formed to hold the pipe section slidably along a longitudinal extension direction (106) of the pipe section (105).

4. Pipe holder according to one of the claims 1 to 3, wherein the fixing section (101) is integrally formed with the pipe holder section (103), wherein the pipe holder is formed particularly as an edged sheet metal.

5. Pipe holder according to one of the claims 1 to 4, wherein the pipe encompassing structure (123) comprises a galvanically isolating and/or thermally isolating material, particularly plastic, and is arrangeable in such a way that the holded pipe section (105) is galvanically and/or thermally isolated from the pipe holder section (103).

6. Pipe holder according to one of the claims 1 to 5, wherein the two straight edges (117, 119) are parallel.

7. Pipe holder according to claim 6, wherein the two straight edges (117, 119) are connected by a bent edge section (121) which is arranged closer to the fixing section (101) than the two straight edges (117, 119).

8. Pipe holder according to claim 6 or 7, wherein the two straight edges (117, 119) are formed in the pipe holder by a recess (115), wherein the pipe section (105) is holdable within the recess (115).

9. Pipe holder according to claim 8, further comprising a locking element (127) for closing the recess.

10. Pipe holder according to the claims 1 to 9, wherein the groove (133) is circular.

11. Pipe holder according to one of the preceding claims, wherein a face of the fixing section (101) is edged relatively to a face of the pipe holder section (103), particularly 90°.

12. Pipe holder according to one of the preceding claims, which is formed for the holding of four pipe sections (105) which are spaced to each other along a second direction (107), wherein the second direction is aligned transverse, particularly perpendicular, to the first direction (125) and transverse, particularly perpendicular, to the longitudinal extension direction (106) of the respective pipe section (105).

13. Fermenter (540) for fermenting of biological material in an internal space (541) of the fermenter, comprising:
a fermenter wall (545), particularly with a circular cross-section, for delimiting the internal space (541);
a pipe holder (500) according to one of the claims 1 to 12, which is attached to an internal side of the fermenter wall (545) by the fixing section of the pipe holder; and
at least one pipe section (505) which is holded by the pipe holder section of the pipe holder,
wherein the pipe section is arranged for heating the internal space (541) of the fermenter and wherein the first direction (125) is aligned along a horizontal direction, particularly a radial direction of the fermenter.

14. Fermenter according to claim 13, wherein a weight of the pipe section is carried on one of the two edges by way of the pipe encompassing structure which is slidably accommodated between the two straight edges.

## Revendications

1. Système de support de tuyau (100, 500), présentant :
une section de fixation (101) servant à fixer le système de support de tuyau au niveau d'une structure de base (102) ;
une section de système de support du tuyau (103) servant à supporter une section de tuyau (105, 505),
sachant que le système de support de tuyau est configuré pour supporter la section de tuyau de manière à permettre un coulissement par rapport à la section de fixation au moyen de la section de support de tuyau, sachant que le système de support de tuyau est configuré pour supporter la section de tuyau de manière à permettre un coulissement le long d'une première direction (125, 125a, 125b), laquelle est orientée de manière transversale par rapport à une direction d'extension longitudinale (106) de la section de tuyau (105),
sachant que le système de support de tuyau présente en outre une structure enveloppant le tuyau (123), cette dernière étant configurée pour envelopper la section de tuyau et pour pouvoir être disposée de manière à pouvoir coulisser dans la section de support de tuyau (103),
sachant que la section de support de tuyau (103) présente deux arêtes (117, 119) droites, qui sont orientées le long de la première direction (125), sachant que la structure enveloppant le tuyau (123) peut être logée entre les deux arêtes de telle manière que la structure enveloppant le tuyau peut glisser le long des deux arêtes (117, 119) droites,
sachant que la structure enveloppant le tuyau (123) présente un profilé de section transversale doté d'une rainure (133), sachant que les deux arêtes (117, 119) droites reçoivent la rainure (133) de manière à pouvoir glisser de telle manière que la structure enveloppant le tuyau (123) peut glisser le long de la première direction (125), le long des deux arêtes (117, 119) droites et que la structure enveloppant le tuyau (123) est empêchée de se déplacer le long d'une direction autre que la première direction,
sachant que la structure enveloppant le tuyau (123) est configurée sous la forme d'un anneau, qui présente dans une zone médiane dans la section transversale la rainure (133), qui s'étend aussi bien tout autour de l'anneau qu'autour d'un axe longitudinal de la section de tuyau lorsque la section de tuyau est logée dans l'anneau.

2. Système de support de tuyau selon la revendication 1, sachant que la première direction (125, 125a, 125b) est orientée de manière perpendiculaire par rapport à une direction d'extension longitudinale (106) de la section de tuyau (105).

3. Système de support de tuyau selon la revendication 1 ou 2, qui est configuré pour supporter la section de tuyau de manière à permettre un coulissement le long de la direction d'extension longitudinale (106) de la section de tuyau (105).

4. Système de support de tuyau selon l'une quelconque des revendications 1 à 3, sachant que la section de fixation (101) est réalisée d'un seul tenant avec la section de support de tuyau (103), sachant que le système de support de tuyau est réalisé en particulier sous la forme d'une tôle pliée.

5. Système de support du tuyau selon l'une quelconque des revendications 1 à 4, sachant que la structure enveloppant le tuyau (123) présente un matériau isolant galvaniquement et/ou thermiquement, en particulier du plastique, et peut être disposée de telle manière que la section de tuyau (105) supportée est isolée galvaniquement et/ou thermiquement de la section de support de tuyau (103).

6. Système de support de tuyau selon l'une quelconque des revendications 1 à 5, sachant que les deux arêtes (117, 119) droites sont parallèles.

7. Système de support de tuyau selon la revendication 6, sachant que les deux arêtes (117, 119) droites sont reliées par l'intermédiaire d'une section d'arête (121) incurvée, laquelle est disposée davantage à proximité de la section de fixation (101) que les deux arêtes (117, 119) droites.

8. Système de support de tuyau selon la revendication 6 ou 7, sachant que les deux arêtes droites sont formées dans le système de support de tuyau au moyen d'un évidement (115), sachant que la section de tuyau (105) peut être supportée à l'intérieur de l'évidement (115).

9. Système de support de tuyau selon la revendication 8, présentant en outre un élément de fermeture (127) servant à fermer l'évidement.

10. Système de support de tuyau selon l'une quelconque des revendications 1 à 9, sachant que la rainure (133) présente une forme circulaire.

11. Système de support de tuyau selon l'une quelconque des revendications précédentes, sachant qu'une surface de la section de fixation (101) est pliée par rapport à une surface de la section de support de tuyau (103), en particulier de 90°.

12. Système de support de tuyau selon l'une quelconque des revendications précédentes, qui est configuré pour supporter quatre sections de tuyau (105) qui sont espacées les unes des autres le long d'une deuxième direction (107), sachant que la deuxième direction est orientée de manière transversale, en particulier de manière perpendiculaire, par rapport à la première direction (125) et de manière transversale, en particulier de manière perpendiculaire, par rapport à la direction d'extension longitudinale (106) de la section de tuyau (105) respective.

13. Fermenteur (540) servant à la fermentation d'une matière biologique dans un espace intérieur (541) du fermenteur, présentant :
une paroi de fermenteur (545), en particulier dotée d'une section transversale de forme circulaire, servant à délimiter l'espace intérieur (541) ;
un système de support de tuyau (500) selon l'une quelconque des revendications 1 à 12, qui est apposé, au moyen de la section de fixation du système de support de tuyau, au niveau d'un côté intérieur de la paroi de fermenteur (545) ; et
au moins une section de tuyau (505), qui est supportée au moyen de la section de support de tuyau du système de support de tuyau,
sachant que la section de tuyau est disposée pour chauffer l'espace intérieur (541) du fermenteur et sachant que la première direction (125) est orientée le long d'une direction horizontale, en particulier d'une direction radiale du fermenteur.

14. Fermenteur selon la revendication 13, sachant qu'un poids de la section de tuyau pèse sur une des deux arêtes droites, par l'intermédiaire de la structure enveloppant le tuyau, qui est logée entre les deux arêtes droites de manière à pouvoir glisser.
